# EUROPEAN PATENT APPLICATION

(11) **EP 0 568 148 A1**
(43) Date of publication of application: **03.11.1993**
(21) Application number: 93201164.6
(22) Date of filing: 21.04.1993
(51) Int. Cl.: A61B 5/11

(54) **Device for testing a joint**

(30) Priority: 21.04.1992 NL 9200721
(71) Applicant: GATSO SPECIAL PRODUCTS B.V., NL-2501 EE Overveen (NL)
(72) Inventor: Gatsonides, Tom, NL-2116 EH Bentveld (NL)
(74) Representative: Bartelds, Erik

(57) **Abstract**

The invention provides a device for testing a joint, in particular a knee joint, comprising means for holding (1,2) body parts connected by the joint for testing, means for connecting (14,15) to one of the body parts for applying a force to the joint and for measuring (11) the displacement occurring in the joint, which means comprise a reference part, which is provided with a reference arm (24), and an indicator arm (25) movably connected to the reference part, wherein the reference part can be fixed to the body part with interposing of at least one connecting member (15) provided with force limiting means, wherein the connecting member (15) is formed by bracket parts (18,19) hingedly connected to each other, the free extremity of one of which has a so-called slip coupling (34).

## Description

The invention relates to a device for testing a joint, in particular a knee joint.

For testing of for instance knee joints use is made of a method wherein the movement in the knee joint is measured using the shin-bone (tibia). For this purpose a force is applied to the shin-bone, while at the same time the kneecap is stabilized. The movement of the shin-bone in relation to the kneecap is a measure of the movement in the knee joint. For application of the said method a device is for instance known which comprises a case for arranging on the shin-bone which is provided with an indicator arm for the shin-bone and a reference arm for the kneecap. Both arms are movable relative to each other and the case. While a stabilizing force is applied to the kneecap an upward respectively downward force is exerted on the shin-bone. The displacement in the knee joint can then be determined on the basis of the reference arms. The case can be fastened round the leg by means of Velcro™ straps. Such a device has the drawback that the fixing of the case on the leg is not optimal, whereby the measurement cannot be performed accurately and reproducibly.

It is the object of the present invention to provide a device for testing a joint, in particular a knee joint, which is improved relative to the prior art and with which the displacement in the knee joint can be determined accurately and safely.

This is achieved by the invention with a device comprising means for holding body parts connected by the joint for testing, means for connecting to one of the body parts for applying a force to the joint and for measuring the displacement occurring in the joint, which means comprise a reference part and an indicator part movably connected to the reference part, wherein the reference part can be fixed to the body part with interposing of at least one connecting member provided with force limiting means. Through the use of force limiting means, which preferably take the form of a so-called slip coupling, in the connecting member an identical fastening round each calf is obtained. Individual variables in the fixing are hereby excluded and more accurate measurements are possible.

The connecting member is preferably formed by bracket parts hingedly connected to each other, the free extremity of one of which has a so-called slip coupling. The bracket parts are arranged round a limb, for example the leg, namely on the proximal calf, whereafter the parts are mutually connected by means of the slip coupling. The slip coupling is herein screwed tight. When a fixed built-in force has been reached the slip coupling will slip whereby further tightening of the slip coupling is made impossible. The fastening of the connecting member round the calf is hereby very constant, whereby variations in the measurements of both left and right knee and of different individuals for testing are prevented.

In a preferred embodiment of the invention the connecting members are connected pivotally to the reference part. The advantage of this is that the positioning of the connecting member can be adapted to any conceivable leg, thereby enabling an optimal fixing of the device.

In order to facilitate determining of the applied force the device is preferably provided with force monitoring means. The reference part preferably has for this purpose an actuating arm connected resiliently thereto, while the force monitoring means comprise at least one microswitch operatively connected to the arm. The microswitch is changed over at a determined force, whereby an indicator means is switched on. Such an indicator means is preferably a visual indicator means, for instance a lamp. By arranging a number of indicator means, wherein the microswitches connected thereto each indicate a determined load, it is possible to determine the load relatively accurately. The first pulling force is preferably very light, whereby damage to the knee joint is prevented. Measurement is possible without risk, even after an acute trauma.

The device further has holding means for stabilizing and supporting the leg. In preference the holding means take the form of support elements and clamping straps adapted to the shape of the body parts for holding. Such holding means prevent displacement of the leg during a measurement and therefore inaccuracies therein.

The present invention will be described further with reference to the annexed drawings, in which:
figure 1 shows a partly cut-away perspective view of a preferred embodiment of a device according to the invention;
figure 2 shows a partly cut-away perspective view of a slip coupling as used in the invention; and
figures 3 and 4 are detail views of the holding means according to the invention.

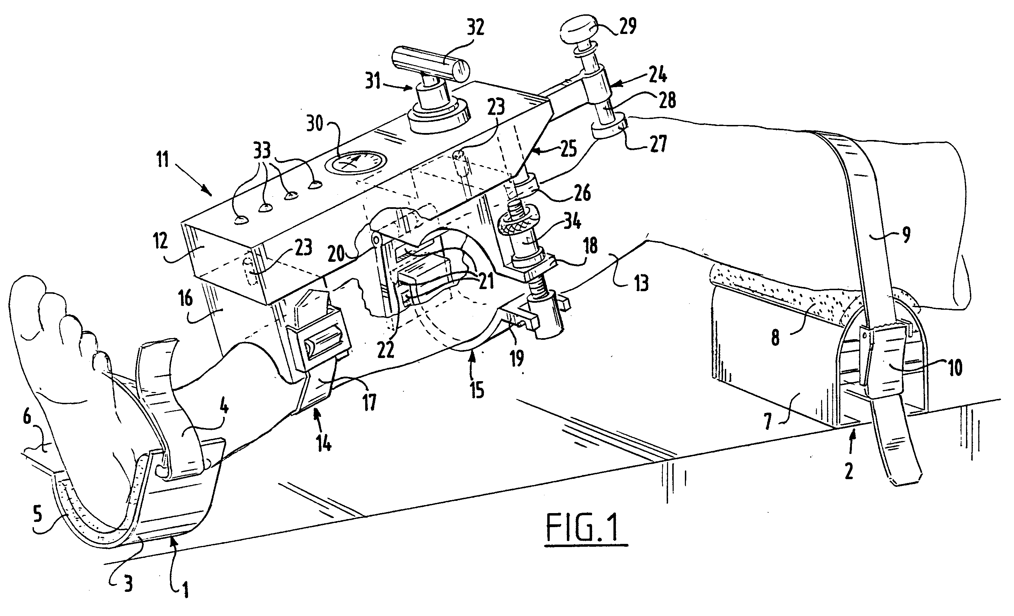

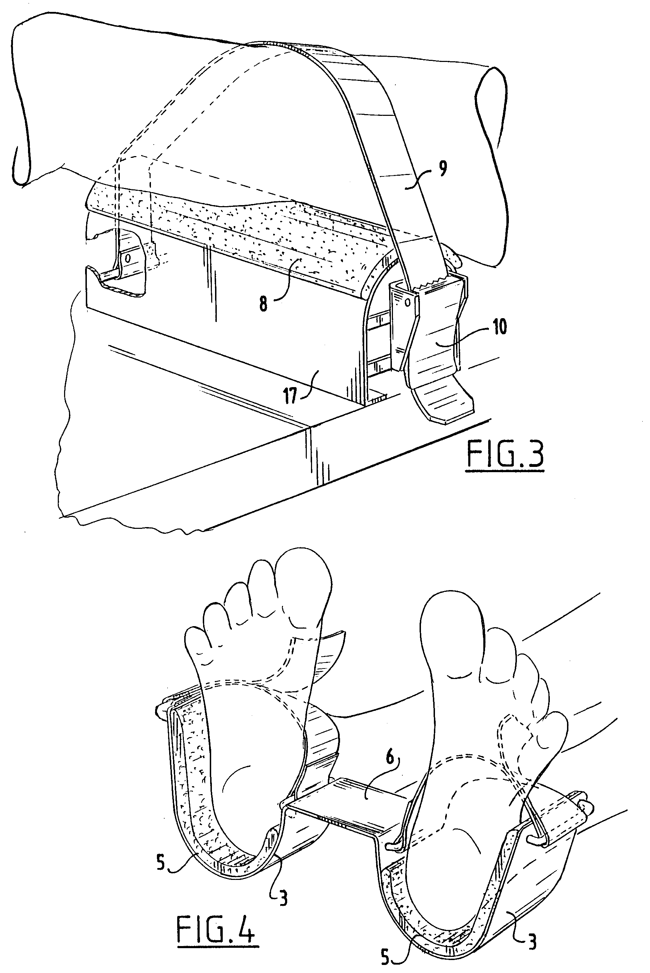

Figure 1 shows the device according to the invention, which is assembled from holding means 1, 2 and a measuring means 11. Holding means 1 comprise footrests 3, which are provided on the upper part with fastening straps 4. The footrests 3 have on the side facing the foot a soft covering 5. The straps 4 are fastenable by means of Velcro™ tape (not visible). Figure 4 shows that the two footrests 3 are mutually connected by means of a bridge 6.

Holding means 2 comprise a support 7, which is provided on its side facing toward the thigh with a soft covering 8. In order to hold fast the leg the means are provided with a fastening strap 9 which can be fixed in any desired position by means of a strap buckle 10 (see also figure 3).

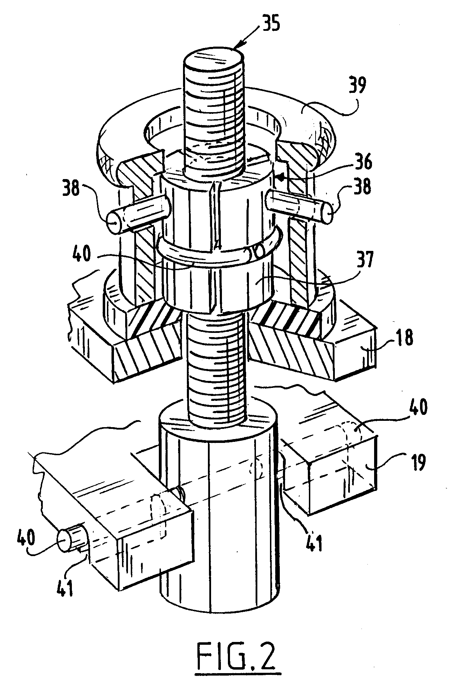

Figure 1 further shows the measuring means 11 of an embodiment of a device according to the invention. The measuring means 11 comprises inter alia a case 12 which is placed on a leg 13 with interposing of two connecting members 14 and 15. Connecting member 14 consists of a block 16 which is adapted to the shape of the leg and which is fastened to the leg 13 by a fastening belt 17. The second connecting member 15 consists of two bracket parts 18 and 19 which are provided with a semi-circular recess and which are mutually connected at one outer end by means of a hinge 20. Hinge 20 is formed such that the diameter of the bracket assembled from the bracket parts 18, 19 can be adapted to the leg for testing. The hinge 20 fixed to bracket part 18 has for this purpose three slots 21 into which a protrusion 22 provided on one extremity of bracket part 19 can be hooked. The bracket parts are coupled at their other end with interposing of a so-called slip coupling (see also figure 2). Both connecting members 14 and 15 are pivotally arranged in the case with interposing of pivot pins 23. The advantage hereof is that the position of the connecting members can be adapted to any random leg for testing.

The measuring means 11 is provided with a reference arm 24 and an indicator arm 25 (only partially visible) which are both arranged pivotally in the case 12. The indicator arm is provided with an indicator member 26. The reference arm 24 consists of a pad 27 for placing on the kneecap and a knob 29 connected thereto by means of a rod-like element 28. By pressing on knob 29 the kneecap is stabilized whereby it can serve as reference. In the rest position, that is, prior to applying a force on the shin-bone, the reference arm 24 and the indicator arm 25 have a determined mutual starting position. A calibrated scale 30 is set to zero on the basis of this position. When a force is applied to the shin-bone by means of handgrip 32 of the force applying means 31, the position of the indicator arm 25 will change. This change is representative of the displacement in the knee joint and is indicated on the calibrated scale 30.

By pulling on the handgrip 32 an upward force is applied to the shin-bone, while a downward force is applied when the handgrip is pressed. Depending on the magnitude of the exerted force one or more microswitches (not shown) in the case are thrown, whereby one or more of the visual indicator means 33, for instance lamps, will light up. The number of lighted lamps gives an indication of the force applied to the shin-bone.

The slip coupling 34 shown in detail in figure 2 consists of a screw 35 which is screwed into a nut 36 consisting of three segments 37 held together by means of a flexible ring 40. The nut is in turn received in a sleeve 39 with interposing of pins 38 connected to the nut segments 37. Sleeve 39 serves to screw fasten the slip coupling after it has been hooked into a recess 41 in bracket part 19 by means of pins 40. In the case the slip coupling is tightened too much a force is applied to the sleeve 39 such that the nut segments 37 are moved outward whereby the inner diameter of the nut increases and the coupling slips. Sleeve 39 serves for tightening of the slip coupling.

The invention provides a device for testing a joint, in particular a knee joint, with which overloading of the knee joint can be prevented during testing. The invention also provides a device which can be arranged in a manner comfortable for the patient and which in addition can be adapted to the shape of the leg.

## Claims

1. Device for testing a joint, in particular a knee joint, comprising means (1;2) for holding body parts connected by the joint for testing, means for connecting to one of the body parts for applying a force to the joint and for measuring the displacement occurring in the joint, which means comprise a reference part (11), which is provided with a reference arm (24), and an indicator arm (25) movably connected to the reference part (11), wherein the reference part (11) can be fixed to the body part with interposing of at least one connecting member (14) provided with force limiting means (34).

2. Device as claimed in claim 1, **characterized in that** the connecting member (14) is formed by bracket parts (18; 19) hingedly connected to each other, the free extremity of one of which has a so-called slip coupling (34).

3. Device as claimed in claim 1 or 2, **characterized in that** the connecting member (14;15) is connected pivotally to the reference part (11).

4. Device as claimed in claim 1, 2 or 3, **characterized in that** the reference part (11) has force monitoring means.

5. Device as claimed in claim 4, **characterized in that** the reference part (11) has an actuating arm connected resiliently thereto and the force monitoring means comprise at least one microswitch operatively connected to the arm.

6. Device as claimed in claim 5, **characterized in that** the force monitoring means comprise a number of switches, each of which is incorporated in the electrical circuit of a visual indicator means (33).

7. Device as claimed in any of the foregoing claims, **characterized in that** the holding means (1;2) comprise a number of support elements (3;7) and clamping straps (4;9) adapted to the shape of the body parts for holding.
